# EUROPEAN PATENT SPECIFICATION

(11) **EP 1 859 750 B1**
(45) Date of publication and mention of the grant of the patent: **12.08.2009**
(21) Application number: 06010709.1
(22) Date of filing: 24.05.2006
(51) Int. Cl.: A61B 17/68, A61B 17/88, A61B 17/92, A61B 17/86

(54) **Locking nail system for arthrodesis reconstruction in calcaneus fractures**
Vorrichtung mit einem Verriegelungsnagel zur Behandlung von Kalkaneusfrakturen
Système avec clou de verrouillage pour le traitement par arthrodèse des fractures du calcaneus

(43) Date of publication of application: 28.11.2007
(73) Proprietor: López-Oliva Munoz, Felipe, 28290 Madrid (ES)
(72) Inventor: López-Oliva Munoz, Felipe, 28290 Madrid (ES)
(74) Representative: Koepe, Gerd L.

(56) References cited:
- ES-A1- 2 164 543
- US-A- 4 913 137
- US-A- 5 855 579
- US-B1- 6 197 029
- US-B1- 6 579 293

## Description

### Object of the invention.

The principal object of the locking nail system for arthrodesis reconstruction of calcaneus fractures is the reconstruction of severe comminuted fractures of the calcaneus in humans and fixation of this bone after reconstructing its principal form, to the talus, in order to attain arthrodesis or fusion with the latter.

### Background to the invention.

Chronic residual pain is the most common sequela following comminuted talar fracture of the calcaneus. There may be multiple causes of this problem: compartment syndrome, reflex-sympathetic dystrophy, plantar cushion syndrome, tarsal and peroneal canal syndrome and subtalar arthritis. This latter cause has been found to be responsible for the majority of poor long-term results of current treatment of these fractures.

Talar fractures account for 8-10% of tarsal tunnel syndrome, particularly in the lateral plantar nerve branch, and for 78% of peroneal nerve entrapment (external submalleolar pain). In 70% of these severe fractures there is chronic pain caused by the posterior subtalar joint, which normally becomes ankylosed after trauma.

Although the latest publications support the results of open reduction and internal fixation of calcaneus fractures, orthopaedic treatment still has many defenders. In other cases, the adoption of an initially conservative philosophy is chosen, treating sequelae if they appear. Like Barnard and Odegard, we believe that perfect restoration of the subtalar joint is impossible.

Numerous arthrodesis techniques have been disclosed for the treatment of post-traumatic subtalar arthritis, including prosthetic replacement. In other cases, triple arthrodesis has been advocated over isolated subtalar fusion, in order to improve foot biomechanics and to prevent degeneration of adjacent joints. According to different reviews conducted in hospitals, isolated arthrodesis of the subtalar joint does not cause degeneration of adjacent joints, even after many years of evolution.

This experience has led to the belief that the old postulations of Stulz and Noble may provide the key to a fracture that has yet to be resolved. Since the subtalar joint cannot be surgically reconstructed in the most severe fractures, and always degenerates, the solution would be to arthrodese it immediately during the acute period in order to prevent the disabling sequelaes that appear during later evolution.

But in addition to fusing the damaged joint, an attempt should be made to reconstruct the form of the hindfoot as far as possible, in order to avoid other associated complications such as compartment syndrome, reflex-sympathetic dystrophy, plantar cushion syndrome and tarsal and peroneal canal syndromes, that would clearly benefit from fracture reduction.

The Spanish patent with publication number ES 2164543, presents a nail that is locked by means of two screws. The preamble of appended claim 1 is based on this disclosure. There are problems inserting this nail into the bone because of its rectangular wedge-shaped geometry and there are resistance problems since it has a very small section in the distal zone that also limits the securing of the lower screw.

### Description of the invention.

In order to alleviate the aforementioned problems, this new locking nail system is presented for arthrodesis reconstruction of calcaneus fractures, which is the object of the present invention patent.

Hence, the invention relates to a locking nail system for arthrodesis reconstruction of calcaneous structures as defined in appended claim 1, comprising a nail, two calcaneous-talar screws, a guide, an applicator guide and two guide tubes, wherein the nail has a blunt end having a threaded hole in order to incorporate the applicator guide; said nail having two through holes through which the calcaneous-talar screws can be positioned in order to arthrodese the subtalar joint; the calcaneous-talar screws are cannulated in order to be guided by the guide tubes, and they are threaded at the distal end for fixation in the spongy bone of the talus; the guide comprises a principal support that has a guide hole at the top, said guide hole having a geometry mating to the exterior geometry of the nail, in order to introduce the nail by means of the applicator guide, and at the bottom the guide has a wad with two holes through which the guide tubes can be passed to permit the calcaneous-talar screws to be inserted in the calcaneous-talus; wherein the nail is a cylindrical geometrical part with a tapered point, and wherein the guide has a grip that is connected to a jaw adapted to be attached to a guide pin placed in the talus, said guide pin defining a reference axis which is transverse to a plane defined by the axis of the guide hole and the axis of one of the holes of the wad, said guide pin serving as a support to adjust the position of the nail and the calcaneous-talar screws by means of a handle, that is situated at the end of the grip.

Preferred embodiments of the invention are now claimed in the dependent claims 2 to 12.

The system consists of the layout of a cylindrical nail with two stabilising fins, a tapered point and two threaded holes, that is inserted into the greater tuberosity of the calcaneus. Likewise, it consists of two cannulated screws that are screwed into the nail holes, inserting them in the talus until they are locked in the nail, thus stabilising the whole assembly, increasing its rigidity and resistance to loads to which it will be subjected.

The use of cannulated screws for arthrodesis of the subtalar joint is a known and implanted technique that offers amongst the best results for the treatment of this type of fracture. The locking nail system for arthrodesis reconstruction in calcaneus fractures, which is the object of the present invention patent, offers a marked improvement in the technique that is habitually employed, because of the insertion of a nail that both supports and locks the said screws, thus making the system more stable and secure, and preventing failure from screw migration. In this way, the foot recovers its natural anatomy, repairing the relationship between the calcaneus and the talus and thus recovering its biomechanical function.

### Description of drawings.

In order to complement this description, and with the aim of contributing to a greater comprehension of the characteristics of the invention, this specification is accompanied by a series of drawings that form an integral part of the same. These drawings are of an illustrative and not limiting nature, and show the following:

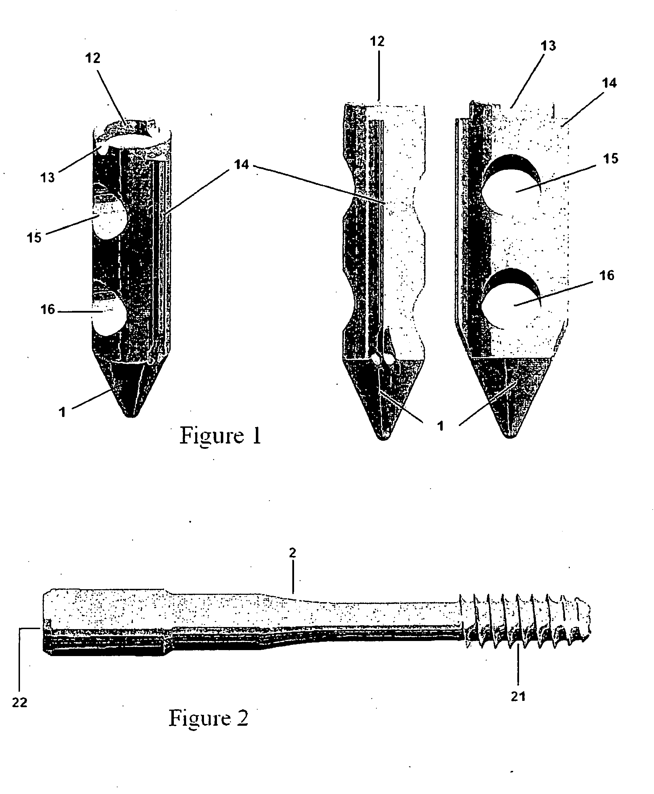

- Figure 1: View of the locking nail.
- Figure 2: View of the cannulated screw.

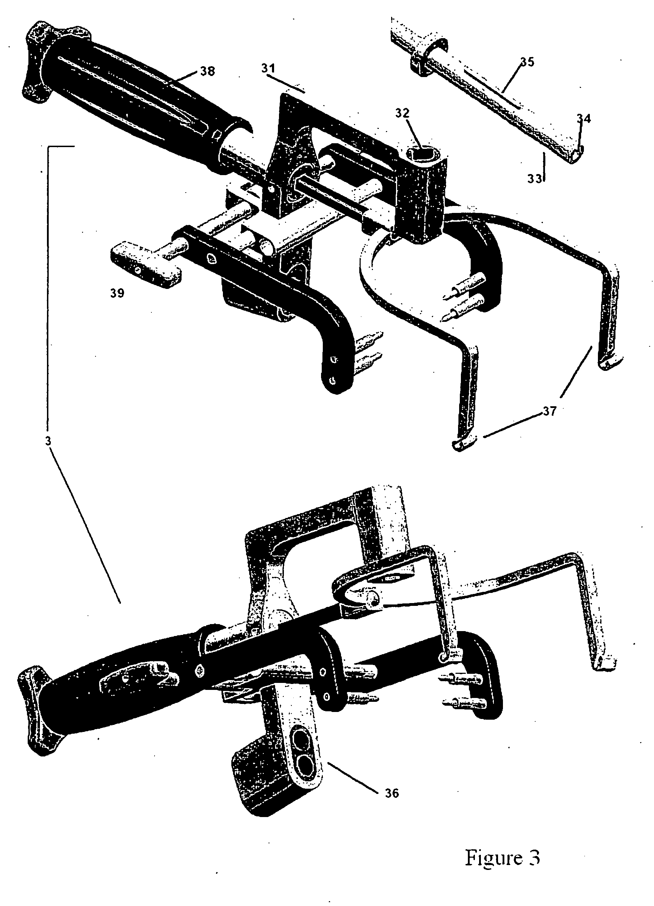

- Figure 3: View of the insertion guide, including the nail applicator guide.

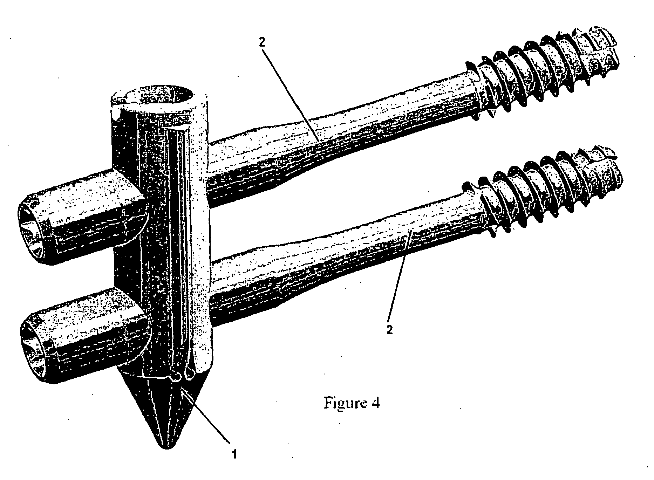

- Figure 4: View of nail with the screws inserted.

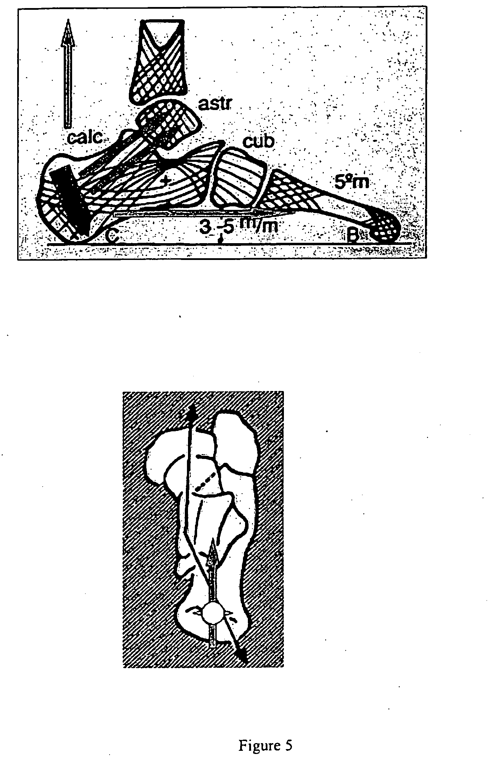

- Figure 5: Diagram of the foot with the locking nail system inserted.

### Preferred embodiment of the invention.

As can be observed in the attached figures, the locking nail system for arthrodesis reconstruction in calcaneus fractures is comprised of the following components: nail (1), screws (2) calcaneus-talar guide (3), guide tubes, bit, depth gauge, cannulated screwdriver, diapason impactor, bit with limiter and reference guide-pin.

The nail (1) is a cylindrical geometrical part with a tapered point, in order to facilitate insertion. Its blunt end has a threaded hole (12) and a groove (13) in order to incorporate an insertion guide (33). Likewise, it has two lateral fins (14) to stabilise the implant and prevent it from rotating. The nail (1) has two through holes (15 and 16) that are partially threaded and set at an angle of 10° to the horizontal shaft, in order to arthrodese the subtalar joint by means of the screws (2).

The calcaneus-talar screws (2) are cannulated (22) in order to insert them with the guide tubes. They are threaded (21) at the distal end for fixation in the spongy bone of the talus. The tip is self-perforating and self-threading in order to facilitate its insertion by means of an optimum torque. The head has a metal thread for fixing it in the nail (1) and it has a hexalobe-type connection that guarantees a more efficient turning action. Preferential length of the screws (2) is not less than 60 mm and not greater than 90 mm.

The guide (3) is an instrument that is used to place the different components in the system in situ, and it is comprised of a principal support (31), that has a guide hole (32) at the top, with the exterior geometry of the nail (1), in order to introduce the same by means of an applicator guide (33), which has a projection (34) at the end that fits into the recess (13) of the nail (1), such that its fixation is guaranteed, preventing rotation movements. Furthermore, the applicator guide (33) has a longitudinal cotter (35) that permits placing the nail (1) at a good angle in the calcaneus, through the guide hole (32).

The lower part of the guide (3) has a wad with two holes (36) that are placed at a 10° angle with respect to the horizontal shaft, in order to pass through the whole system of cannulas and instruments that permit the screws (2) to be placed in the calcaneus-talus.

The guide (3) has a grip (38) that, in addition to serving as such, is connected to the jaw (37) that is attached to a pin that has been previously placed in the talus and that serves as a reference axis and as a support to adjust the position of the nail (1), by means of a handle (39) that is situated at the end of the grip (38).

The guide tubes must be embodied in duplicate except for the trocar tip, permitting suitable guidance and direction of the different instruments used to prepare the bed and position the screws (2). The instruments used to prepare the bed are the guide-pin, the cannulated bit with a diameter corresponding to the nucleus of the screws (2), and the screws themselves (2). Therefore, three guide tubes of different diameters are necessary, and the trocar tip. Other elements used by the system are the depth gauge to determine the length of the screws (2), the cannulated screwdriver to insert the screws (2), the diapason impactor to strike the nail (1), the bit with limiter to work the nail bed (1) and the reference pin for the guide support (3).

The implantation of the locking nail system for arthrodesis reconstruction in calcaneus fractures, object of the present invention patent, is embodied as follows:

First, and after opportune surgical procedures, the guide-pin is placed in the centre of the talar head. The guide-pin is the spatial reference for the nail instrumentation (1). It is placed from the internal face of the foot, locating the centre of talar head by radiological means. The guide-pin should be positioned on the frontal plane parallel to the ankle joint interline, and on the axial plane, perpendicular to the foot shaft. Surgery should not be continued until it is verified that the guide-pin is in the correct position.

The guide (3) is initially supported by the guide-pin situated in the talar head, and then the guide (3) is initially adjusted to the greater tuberosity using the handle (39) situated at the end of the grip (38).

A radiological control is performed on the position, using guide-pins to simulate the direction of the screws (2) by inserting them in the holes (36) made for this purpose in the guide arms (3). These pin-guides will indicate the position of the definite screws (2). If they do not sit appropriately in the talar head, the guide (3) will have to be re-positioned.

At this stage, the guide permits the calcaneus axis to be distracted using the handle (39) and grip (38) and it also permits flexion-extension movements to be applied to the same, by mobilising the guide (3), using the guide-pin placed in the talar head as an axis. Then, a hole is drilled to house the nail (1) in the greater tuberosity. If the guide (3) is in the correct position, drilling of the greater tuberosity is not a problem. Drilling should continue as far as the bit limiter. The only precaution to take is to protect the Achiles tendon adequately.

In some cases, the severity of the comminuted fracture means that drilling will be effected on fractured bone. This situation does not pose any disadvantage for inserting the implant as the latter does not require intact bone for correct functioning. When the bit is removed, it should continue to revolve in order to extract bone dislodged during drilling. The drilling direction should not be inverted. In the majority of cases, this bone will be sufficient for the graft in the subtalar arthrodesis.

The nail (1) is held by the guide (3) and is placed in the previously marked channel. The nail (1) is inserted by hammering. The guide (3) will direct the nail towards the lower zone in the greater tuberosity where it will be inserted in the plantar fascia. The nail (1) must be completely inserted up to the applicator limiter, thus attaining correct alignment of the holes of the screws (2) and the guide (3) that applies the same.

With the guide tubes in position on the guide (3), the surgeon makes a small incision reaching the bone. The guide-pin is inserted. The length of the screws (2) is measured with the depth gauge. After the guide-pin, the 4.5 mm cannulated bit is inserted. The screws (2) are then positioned. When the system is in position, the bone extracted from drilling the nail (1) is applied.

Having sufficiently disclosed the nature of the present invention, as well as the practical embodiment of the same, the only thing to add is that said invention may undergo certain variations with respect to form and materials, provided that such alterations do not substantially change the characteristics that are claimed below.

## Claims

1. A locking nail system for arthrodesis reconstruction of calcaneous structures, comprising a nail (1), two calcaneous-talar screws (2), a guide (3), an applicator guide (33) and two guide tubes, wherein
- the nail (1) has a blunt end having a threaded hole (12) in order to incorporate the applicator guide (33); said nail (1) having two through holes (15, 16) through which the calcaneous-talar screws (2) can be positioned in order to arthrodese the subtalar joint;
- the calcaneous-talar screws (2) are cannulated (22) in order to be guided by the guide tubes, and they are threaded (21) at the distal end for fixation in the spongy bone of the talus;
- the guide (3) comprises a grip (38) and a principal support (31) that has a guide hole (32) at the top, said guide hole (32) having a geometry mating to the exterior geometry of the nail (1), in order to introduce the nail (1) by means of the applicator guide (33), and at the bottom the guide (3) has a wad with two holes (36) through which the guide tubes can be passed to permit the calcaneous-talar screws to be inserted in the calcaneous-talus;
**characterized in that**,
the nail (1) is a cylindrical geometrical part with a tapered point, and **in that** the grip (38) of the guide (3) is connected to a jaw (37) adapted to be attached to a guide pin placed in the talus, said guide pin defining a reference axis which is transverse to a plane defined by the axis of the guide hole (32) and the axis of one of the holes (36) of the wad, said guide pin serving as a support to adjust the position of the nail (1) and the calcaneous-talar screws (2) by means of a handle (39), that is situated at the end of the grip (38).

2. The locking nail system according to claim 1, wherein the blunt end of the nail (1) has a recess (13) capable of incorporating the applicator guide (33).

3. The locking nail system according to claim 1 or claim 2, wherein the nail (1) has at least one, preferably two lateral fins (14).

4. The locking nail system according to any of claims 1 to 3, wherein the two through holes (15, 16) of the nail (1) are partially threaded and/or set an angle of 10° to the horizontal shaft.

5. The locking nail system according to any of claims 1 to 4, wherein the tips of the calcaneous-talar screws (2) are self-perforating and self threading.

6. The locking nail system according to any of claims 1 to 5, wherein the calcaneus-talar screws (2) have heads, said heads having a metal thread for fixing it in the nail (1) and preferably having a hexalobe-type connection.

7. The locking nail system according to any of claims 1 to 6, wherein the length of the calcaneus-talar screws (2) is not less than 60 mm and not greater than 90 mm.

8. The locking nail system according to any of claims 1 to 7, which system further comprises one or more of the following elements: bit, depth gauge, cannulated screwdriver, diapason impactor, bit with limiter and reference guide-pin.

9. The locking nail system according to claim 8, wherein the depth gauge is capable of the determining the length of the calcaneus-talar screws (2) and/or the cannulated screwdriver is capable of inserting the calcaneus-talar screws (2) and/or the diapason impactor is capable of striking the nail (1) and/or the bit with limiter is capable of working the bed of the nail (1) and/or the reference guide pin is capable of serving for guide support

10. The locking nail system according to any of claims 1 to 9, wherein the through holes (15, 16) of the nail (1) permit the subtalar joint to be arthrodesed by means of the calcaneus-talar screws (2).

11. The locking nail system according to any of claims 1 to 10, wherein the applicator guide (33) has a projection (34) at the end that fits into the recess (13) of the nail (1), such that its fixation is guaranteed, preventing rotation movements.

12. The locking nail system according to any of claims 1 to 11, wherein the applicator guide (33) has a longitudinal cotter (35) capable of mating with a slot in the guide hole (32) for a placement of the nail (1) in the calcaneus.

## Patentansprüche

1. Einen Verschlussnagel umfassendes System für eine Arthrodese-Rekonstruktion von Calcaneus-Strukturen, umfassend einen Nagel (1), zwei Calcaneus-Talus-Schrauben (2), eine Führung (3), eine Applikator-Führung (33) und zwei Führungsrohre, worin
- der Nagel (1) ein stumpfes Ende mit einem, mit einem Gewinde versehenen Loch (12) aufweist, um die Applikator-Führung (33) zu integrieren; und der Nagel (1) zwei durchgehende Löcher (15, 16) aufweist, durch die die Calcaneus-Talus-Schrauben (2) positioniert werden können, um das Subtalus-Gelenk zu arthrodesieren;
- die Calcaneus-Talus-Schrauben (2) kanüliert sind (22), um von den Führungsrohren geführt zu werden, und sie sind mit einem Gewinde (21) am distalen Ende zur Fixierung in dem porösen Knochen des Talus versehen;
- die Führung (3) einen Griff (38) und einen Hauptträger (31) umfasst, der ein Führungsloch (32) auf der Oberseite aufweist, wobei das Führungsloch (32) eine Geometrie hat, die zu der Außengeometrie des Nagels (1) passt, um den Nagel (1) mittels der Applikator-Führung (33) einzuführen, und auf der Unterseite weist die Führung (3) einen Vorstoß mit zwei Löchern (36) auf, durch die die Führungsrohre hindurchgeführt werden können, um zu erlauben, dass die Calcaneus-Talus-Schrauben in den Calcaneus-Talus eingesetzt werden;
**dadurch gekennzeichnet, dass**
der Nagel (1) ein zylindrisches geometrisches Teil mit einer konischen Spitze ist, und dass
der Griff (38) der Führung (3) mit einer Klaue (37) verbunden ist, die dafür angepasst ist, an einem Führungsstift befestigt zu werden, der in dem Talus platziert ist, wobei der Führungsstift eine Referenz-Achse definiert, die quer zu einer Ebene ist, die durch die Achse des Führungslochs (32) und die Achse eines der Löcher (36) des Vorstoßes definiert ist, wobei der Führungsstift als Stützte dient, um die Position des Nagels (1) und der Calcaneus-Talus-Schrauben (2) mittels eines Einstellgriffs (39) einzustellen, der am Ende des Griffs (38) gelegen ist.

2. Einen Verschlussnagel umfassendes System nach Anspruch 1, worin das stumpfe Ende des Nagels (1) eine Aussparung (13) aufweist, die in der Lage ist, die Applikator-Führung (33) zu integrieren.

3. Einen Verschlussnagel umfassendes System nach Anspruch 1 oder Anspruch 2, worin der Nagel (1) wenigstens einen, vorzugsweise zwei seitliche Flügel (14) aufweist.

4. Einen Verschlussnagel umfassendes System nach irgendeinem der Ansprüche 1 bis 3, wobei die beiden durchgehenden Löcher (15, 16) des Nagels (1) teilweise mit einem Gewinde versehen und/oder in einem Winkel von 10° zu der horizontalen Achse gesetzt sind.

5. Einen Verschlussnagel umfassendes System nach irgendeinem der Ansprüche 1 bis 4, worin die Spitzen der Calcaneus-Talus-Schrauben (2) selbst-perforierend und selbst-einziehend sind.

6. Einen Verschlussnagel umfassendes System nach irgendeinem der Ansprüche 1 bis 5, worin die Calcaneus-Talus-Schrauben (2) Köpfe aufweisen, wobei die Köpfe ein Metall-Gewinde haben, um den Kopf in dem Nagel (1) zu fixieren, und vorzugsweise eine Verbindung des Sechseck-Typs haben.

7. Einen Verschlussnagel umfassendes System nach irgendeinem der Ansprüche 1 bis 6, worin die Länge der Calcaneus-Talus-Schrauben (2) nicht geringer ist als 60 mm und nicht größer ist als 90 mm.

8. Einen Verschlussnagel umfassendes System nach irgendeinem der Ansprüche 1 bis 7, wobei das System weiter eines oder mehrere der folgenden Elemente umfasst: eine Bohrspitze, einen Tiefenmesser, einen kanülierten Schraubenzieher, einen Mensur-Stoßkörper, eine Bohrspitze mit Begrenzer und einen Referenz-Führungsstift.

9. Einen Verschlussnagel umfassendes System nach Anspruch 8, worin der Tiefenmesser in der Lage ist, die Länge der Calcaneus-Talus-Schrauben (2) zu bestimmen, und/oder der kanülierte Schraubenzieher in der Lage ist, die Calcaneus-Talus-Schrauben (2) einzusetzen, und/oder der Mensur-Stoßkörper in der Lage ist, den Nagel (1) einzuschlagen, und/oder die Bohrspitze mit Begrenzer in der Lage ist, das Bett für den Nagel (1) zu schaffen und/oder der Referenz-Führungsstift in der Lage ist, als Führungsstütze zu dienen.

10. Einen Verschlussnagel umfassendes System nach irgendeinem der Ansprüche 1 bis 9, worin die durchgehenden Löcher (15, 16) des Nagels (1) erlauben, dass das Subtalus-Gelenk mittels der Calcaneus-Talus-Schrauben (2) arthrodesiert wird.

11. Einen Verschlussnagel umfassendes System nach irgendeinem der Ansprüche 1 bis 10, worin die Applikator-Führung (33) einen Vorsprung (34) am Ende aufweist, der in die Aussparung (13) des Nagels (1) passt, so dass seine Fixierung garantiert ist und Rotationsbewegungen vermieden werden.

12. Einen Verschlussnagel umfassendes System nach irgendeinem der Ansprüche 1 bis 11, worin die Applikator-Führung (33) einen länglichen Splint (35) aufweist, der in der Lage ist, zu einem Schlitz in dem Führungsloch (32) für ein Platzieren des Nagels (1) in dem Calcaneus zu passen.

## Revendications

1. Système à clou de verrouillage pour la reconstruction par arthrodèse de structures calcanéennes, comprenant un clou (1), deux vis calcanéo-astragaliennes (2), un guide (3), un guide applicateur (33) et deux tubes de guidage, dans lequel
- le clou (1) est pourvu d'une extrémité franche comportant un trou taraudé (12) pour incorporer le guide applicateur (33) ; ledit clou (1) étant pourvu de deux trous de passage (15, 16) à travers lesquels les vis calcanéo-astragaliennes (2) peuvent être positionnées pour réaliser une arthrodèse de l'articulation sous-astragalienne ;
- les vis calcanéo-astragaliennes (2) sont canulées (22) pour être guidées par les tubes de guidage et elles sont filetées (21) au niveau de l'extrémité distale en vue d'une fixation dans l'os spongieux de l'astragale ;
- le guide (3) comprend une poignée (38) et un support principal (31) pourvu d'un trou de guidage (32) à son sommet, ledit trou de guidage (32) ayant une géométrie qui correspond à la géométrie extérieure du clou (1) afin d'introduire le clou (1) au moyen du guide applicateur (33) et le guide (3) est pourvu, en bas, d'un tampon comportant deux trous (36) à travers lesquels les tubes de guidage peuvent être passés pour permettre l'insertion des vis calcanéo-astragaliennes dans le calcanéum et l'astragale ;
**caractérisé en ce que**
le clou (1) est une pièce géométrique cylindrique pourvue d'une pointe conique et **en ce que**
la poignée (38) du guide (3) est connectée à une mâchoire (37) adaptée pour être attachée à une broche de guidage placée dans l'astragale, ladite broche de guidage définissant un axe de référence qui est perpendiculaire à un plan défini par l'axe du trou de guidage (32) et l'axe de l'un des trous (36) du tampon, ladite broche de guidage servant de support pour régler la position du clou (1) et des vis calcanéo-astragaliennes (2) au moyen d'une manette (39) qui est située à l'extrémité de la poignée (38).

2. Système à clou de verrouillage selon la revendication 1, dans lequel l'extrémité franche du clou (1) est pourvue d'une cavité (13) capable d'incorporer le guide applicateur (33).

3. Système à clou de verrouillage selon la revendication 1 ou la revendication 2, dans lequel le clou (1) est pourvu d'au moins une, de préférence de deux ailettes latérales (14).

4. Système à clou de verrouillage selon l'une des revendications 1 à 3, dans lequel les deux trous de passage (15, 16) du clou (1) sont partiellement taraudés et/ou définissent un angle de 10° par rapport à la tige horizontale.

5. Système à clou de verrouillage selon l'une des revendications 1 à 4, dans lequel les pointes des vis calcanéo-astragaliennes (2) sont auto-perforantes et auto-taraudeuses.

6. Système à clou de verrouillage selon l'une des revendications 1 à 5, dans lequel les vis calcanéo-astragaliennes (2) ont des têtes, lesdites têtes étant pourvues d'un filet métallique pour les fixer dans le clou (1) et présentant de préférence une connexion du type hexalobe.

7. Système à clou de verrouillage selon l'une des revendications 1 à 6, dans lequel la longueur des vis calcanéo-astragaliennes (2) n'est pas inférieure à 60 mm et pas supérieure à 90 mm.

8. Système à clou de verrouillage selon l'une des revendications 1 à 7, lequel système comprend, en outre, un ou plusieurs des éléments suivants : une mèche, une jauge de profondeur, un tournevis canulé, un impacteur en diapason, une mèche avec limiteur et une broche de guidage de référence.

9. Système à clou de verrouillage selon la revendication 8, dans lequel la jauge de profondeur est capable de déterminer la longueur des vis calcanéo-astragaliennes (2) et/ou le tournevis canulé est capable d'insérer les vis calcanéo-astragaliennes (2) et/ou l'impacteur en diapason est capable de frapper le clou (1) et/ou la mèche avec limiteur est capable de travailler le logement du clou (1) et/ou la broche de guidage de référence est capable de servir de support de guidage.

10. Système à clou de verrouillage selon l'une des revendications 1 à 9, dans lequel les trous de passage (15, 16) du clou (1) permettent de réaliser une arthrodèse de l'articulation sous-astragalienne au moyen des vis calcanéo-astragaliennes (2).

11. Système à clou de verrouillage selon l'une des revendications 1 à 10, dans lequel le guide applicateur (33) est pourvu, à son extrémité, d'une saillie (34) qui s'adapte dans la cavité (13) du clou (1) de manière à ce que sa fixation soit garantie, empêchant des mouvements de rotation.

12. Système à clou de verrouillage selon l'une des revendications 1 à 11, dans lequel le guide applicateur (33) est pourvu d'une clavette longitudinale (35) capable de s'apparier avec une fente dans le trou de guidage (32) pour une mise en place du clou (1) dans le calcanéum.
